# EUROPEAN PATENT APPLICATION

(11) **EP 3 378 469 A1**
(43) Date of publication of application: **26.09.2018**
(21) Application number: 18163711.7
(22) Date of filing: 23.03.2018
(51) Int. Cl.: A61K 9/10, A61K 47/26, A61K 31/22

(54) **PHARMACEUTICAL COMPOSITION OF SIMVASTATIN OR SALT THEREOF**

(30) Priority: 24.03.2017 GB 201704687
(71) Applicant: Wockhardt UK Ltd, Wrexham LL13 9UF (GB)
(72) Inventor: Wynne, Neil, Wrexham, LL13 9UF (GB); Warnett, Ruth, Wrexham, LL13 9UF (GB)
(74) Representative: Gill, Siân Victoria

(57) **Abstract**

A pharmaceutical composition for oral administration in the form of an aqueous suspension comprising simvastatin or salt thereof, a low hygroscopic excipient; and a buffer, wherein the composition has a pH in the range of about 3.5 to about 4.0. The composition of the present invention contains less than about 1% of Impurity A when stored at about 25°C and about 60% RH for a period of at least 3 months.

## Description

### BACKGROUND OF INVENTION

Simvastatin is an inhibitor of 3-hydroxy-3-methylglutaryl-coenzyme A (HMG-CoA) reductase. This enzyme catalyzes the conversion of HMG-CoA to mevalonate, which is an essential step in the biosynthesis of cholesterol. Simvastatin is butanoic acid, 2,2-dimethyi-,1,2,3,7,8,8a-hexahydro-3,7-dimethyl-8-[2-tetrahydro-4-hydroxy-6-oxo-2H-pyran-2-yl)-ethyl]-1-naphthalenyl ester, [1S-[1α,3α,7β,8β(2S*,4S*),-8aβ]] and belongs to a group of compounds called statins.

Simvastatin is a white/off-white non-hygroscopic crystalline powder and is practically insoluble in water. Simvastatin is marketed as Zocor® tablet in 10 mg, 20 mg, 40 mg and 80 mg strengths.

PCT Publication no. WO2007/125339 A1 discloses simvastatin suspension for oral administration comprising, at least one suspending agent, and at least one preservative. PCT Publication no. WO2010/098906 A1 discloses liquid formulation of simvastatin with solubilizer. PCT Publication no. WO2000/053566 A1 discloses oral suspension of simvastatin with alkaline pH. PCT Publication no. WO2002/100394 A1 discloses oral pharmaceutical composition containing a statin derivative comprising oily excipient and one stabilizing agent.

Many patients, particularly the elderly, experience difficulty in swallowing solid dosage forms like tablets and/or capsules. This has resulted in the widespread practice of crushing tablets and opening capsules. Administering a medicine by crushing or opening is quite cumbersome and may lead to erroneous dosing.

It is advisable to control the levels of impurities in the pharmaceutical compositions and to ensure that the impurity is present in the lowest possible levels. Further, the level of water content of the finished product has a great influence on the stability of the drug, especially the drugs like simvastatin, whose degradation pathway is dominated by the hydrolysis reaction. Therefore, formulating a pharmaceutical composition comprising a simvastatin to obtain a stable pharmaceutical composition with low level of impurities is a challenging task.

There is a need for stable pharmaceutical composition of simvastatin in the form of an aqueous suspension for oral administration while maintaining a suitable bioavailability of drug after oral administration.

### SUMMARY OF THE INVENTION

One aspect of the invention discloses a pharmaceutical composition for oral administration in the form of an aqueous suspension comprising (a) about 0.3% w/v to about 0.9% w/v of simvastatin or salt thereof, and (b) about 0.05% w/v to about 0.2% w/v of surfactant, wherein the composition has a pH in the range of about 3.5 to about 4.0 or in the range of about 3.5 to about 4.5. In another aspect of the invention discloses a pharmaceutical composition for oral administration in the form of an aqueous suspension comprising: (a) about 0.3% w/v to about 0.9% w/v of simvastatin or salt thereof, (b) a low hygroscopic excipient; and (c) a buffer, wherein the composition has a pH in the range of about 3.5 to about 4.0, and the composition contains less than about 1% of Impurity A when stored at about 25°C and about 60% RH for a period of at least 3 months.

In one embodiment, the simvastatin or salt thereof is present in about 0.4% w/v or about 0.8% w/v of the composition. In one embodiment, the pH of the composition ranges from 3.7 to 3.9. In one embodiment, the surfactant is polysorbate. In another embodiment, the polysorbate is polysorbate 80. The composition further comprises a pharmaceutically acceptable excipient selected from preservative, buffer, suspending agent, antioxidant, sweetener, flavour and mixture thereof. The preservative is one or more hydroxybenzoates are selected from methyl hydroxybenzoate, ethyl hydroxybenzoate, propyl hydroxybenzoate, and mixtures thereof. The buffer may be selected from citric acid monohydrate, sodium citrate, glycine, tris, bicine, sodium phosphate, sodium dihydrogen phosphate and mixtures thereof. The suspending agent is selected from aluminium magnesium silicate, sodium carmellose, methyl cellulose, hydroxypropyl methylcellulose, sodium alginate, xanthan gum, and mixtures thereof. In one embodiment, the composition remains stable for at least 1 month, or 2 months, or 3 months when stored at about 25°C and about 60% (RH) relative humidity. In another embodiment, the composition remains stable for at least 1 month, or 2 months, or 3 months when stored at 40°C and 75% (RH) relative humidity.

Another embodiment discloses a pharmaceutical composition for oral administration in the form of an aqueous suspension comprising about 0.3% w/v to about 0.9% w/v of simvastatin or salt thereof, and about 0.05% w/v to about 0.2% w/v of surfactant, wherein the composition has a pH in the range of 3.7 to 3.9.

Another aspect discloses a pharmaceutical composition for oral administration in the form of an aqueous suspension comprising about 0.3% w/v to about 0.9% w/v of simvastatin or salt thereof, and about 0.05% w/v to about 0.2% w/v of polysorbate 80, wherein the composition has a pH in the range of about 3.5 to about 4.0 or in the range of about 3.5 to about 4.5. In one embodiment, the simvastatin or salt thereof is present in about 0.4% w/v or about 0.8% w/v of the composition. In one embodiment, the pH of the composition ranges from 3.7 to 3.9. In another embodiment, the polysorbate 80 is present in about 0.11 % w/v of the composition.

Another embodiment discloses pharmaceutical composition for oral administration in the form of an aqueous suspension comprising: (a) about 0.4% w/v of simvastatin or salt thereof, (b) about 0.11% w/v of polysorbate 80, (c) about 0.72% w/v of aluminium magnesium silicate, (d) about 0.08% w/v of xanthan gum, (e) about 3.50% w/v of propylene glycol, (f) about 70% w/v to about 90% w/v of liquid maltitol and (g) a buffer, wherein the composition has a pH in the range of 3.5 to 3.9 and the composition contains less than about 1% of Impurity A when stored at about 25°C and about 60% RH for a period of at least 3 months.

Further embodiment discloses pharmaceutical composition for oral administration in the form of an aqueous suspension comprising: (a) about 0.8% w/v of simvastatin or salt thereof, (b) about 0.11% w/v of polysorbate 80, (c) about 0.72% w/v of aluminium magnesium silicate, (d) about 0.08% w/v of xanthan gum, (e) about 3.50% w/v of propylene glycol (f) about 70% w/v to about 90% w/v of liquid maltitol and (g) a buffer wherein the composition has a pH in the range of 3.5 to 3.9 and the composition contains less than about 1% of Impurity A when stored at about 25°C and about 60% RH for a period of at least 3 months.

Another embodiment discloses pharmaceutical composition for oral administration in the form of an aqueous suspension comprising: (a) about 0.8% w/v of simvastatin or salt thereof, (b) about 0.11% w/v of polysorbate 80, (c) about 0.72% w/v of aluminium magnesium silicate, (d) about 0.08% w/v of xanthan gum, (e) about 3.50% w/v of propylene glycol (f) about 70% w/v to about 90% w/v of liquid maltitol and (g) a buffer wherein the composition has a pH in the range of 3.5 to 3.9 and the composition contains less than about 0.5% of Impurity B or Impurity C or Impurity D, when stored at about 25°C and about 60% RH for a period of at least 3 months.

One embodiment discloses a pharmaceutical composition for oral administration in the form of an aqueous suspension comprising about 0.3% w/v to about 0.9% w/v of simvastatin or salt thereof, and about 0.05% w/v to about 0.2% w/v of surfactant, wherein the composition has a pH in the range of about 3.5 to about 4, for use in the treatment of hypercholesterolemia.

One embodiment discloses a process of preparing a pharmaceutical composition for oral administration in the form of an aqueous suspension comprising about 0.3% w/v to about 0.9% w/v of simvastatin or salt thereof, about 0.05% w/v to about 0.2% w/v of surfactant and a pharmaceutically acceptable excipient(s), wherein the composition has a pH in the range of about 3.5 to about 4.0 or in the range of about 3.5 to about 4.5, wherein the process comprises the steps of:
(i) adding and mixing the pharmaceutically acceptable excipient(s) in water to obtain a mixture,
(ii) separately adding the surfactant into water with stirring to obtain a dispersion,
(iii) adding gradually simvastatin or salt thereof to the dispersion of step (ii) with mixing,
(iv) adding the dispersion of step (iii) into the mixture of step (i) with mixing to obtain dispersion, and
(v) finally adjusting a volume with water and the pH to obtain the pharmaceutical composition for oral administration in the form of an aqueous suspension.

Another embodiment discloses a process of preparing a pharmaceutical composition for oral administration in the form of an aqueous suspension comprising about 0.3% w/v to about 0.9% w/v of simvastatin or salt thereof, about 0.05% w/v to about 0.2% w/v of polysorbate 80 and a pharmaceutically acceptable excipient(s), wherein the composition has a pH in the range of about 3.5 to about 4.0 or in the range of about 3.5 to about 4.5, wherein the process comprises the steps of:
(i) adding and mixing the pharmaceutically acceptable excipient(s) in water to obtain a mixture,
(ii) separately adding polysorbate 80 into water with stirring to obtain a dispersion,
(iii) adding gradually simvastatin or salt thereof to the dispersion of step (ii) with mixing,
(iv) adding the dispersion of step (iii) into the mixture of step (i) with mixing to obtain dispersion, and
(v) finally adjusting a volume with water and the pH to obtain the pharmaceutical composition for oral administration in the form of an aqueous suspension.

Yet another embodiment discloses a process of preparing a pharmaceutical composition for oral administration in the form of an aqueous suspension comprising about 0.3% w/v to about 0.9% w/v of simvastatin or salt thereof, about 0.05% w/v to about 0.2% w/v of polysorbate 80, and a pharmaceutically acceptable excipient(s), wherein the composition has a pH in the range of 3.7 to 3.9, wherein the process comprises the steps of:
(i) adding and mixing the pharmaceutically acceptable excipient(s) in water to obtain a mixture,
(ii) separately adding polysorbate 80 into water with stirring to obtain a dispersion,
(iii) adding gradually simvastatin or salt thereof to the dispersion of step (ii) with mixing,
(iv) adding the dispersion of step (iii) into the mixture of step (i) with mixing to obtain dispersion, and
(v) finally adjusting a volume with water and the pH to obtain the pharmaceutical composition for oral administration in the form of an aqueous suspension.

Another aspect of the invention discloses a pharmaceutical composition for oral administration in the form of an aqueous suspension comprising about 0.3% w/v to about 0.9% w/v of simvastatin or salt thereof, a low hygroscopic excipient wherein the composition has a pH in the range of about 3.5 to about 4.5.

Another aspect of the invention discloses a pharmaceutical composition for oral administration in the form of an aqueous suspension comprising: (a) about 0.4% w/v of simvastatin or salt thereof, (b) about 0.11% w/v of polysorbate 80, (c) about 0.72% w/v of aluminium magnesium silicate, (d) about 0.08% w/v of xanthum gum, and (e) about 3.50% w/v of propylene glycol, wherein the composition has a pH in the range of 3.7 to 3.9.

Another aspect of the invention discloses a pharmaceutical composition for oral administration in the form of an aqueous suspension comprising: (a) about 0.8% w/v of simvastatin or salt thereof, (b) about 0.11% w/v of polysorbate 80, (c) about 0.72% w/v of aluminium magnesium silicate, (d) about 0.08% w/v of xanthum gum, and (e) about 3.50% w/v of propylene glycol, wherein the composition has a pH in the range of 3.7 to 3.9.

Another aspect of the invention discloses a process of preparing a pharmaceutical composition for oral administration in the form of an aqueous suspension comprising about 0.3% w/v to about 0.9% w/v of simvastatin or salt thereof, about 0.05% w/v to about 0.2% w/v of surfactant and a pharmaceutically acceptable excipient(s), wherein the composition has a pH in the range of about 3.5 to about 4.5, wherein said process comprises steps of:
(i) adding and mixing the pharmaceutically acceptable excipient(s) in water to obtain a mixture,
(ii) separately adding the surfactant into water with stirring to obtain a dispersion,
(iii) adding gradually simvastatin or salt thereof to the dispersion of step (ii) with mixing,
(iv) adding the dispersion of step (iii) into the mixture of step (i) with mixing to obtain smooth dispersion, and
(v) finally adjusting a volume with water and the pH to obtain the pharmaceutical composition for oral administration in the form of an aqueous suspension.

### DETAILED DESCRIPTION OF THE INVENTION

One embodiment discloses a pharmaceutical composition for oral administration in the form of an aqueous suspension comprising simvastatin or salt thereof, and a low hygroscopic excipient, wherein the composition has a pH in the range of about 3.5 to about 4.0 or in the range of about 3.5 to about 4.5.

As used herein the term "pharmaceutical composition" means a mixture containing a therapeutic compound(s) to be administered to a mammal, e.g. a human, in order to prevent, treat or control a particular disease or condition affecting the mammal.

The term "pharmaceutically acceptable excipients" herein relates to non-active pharmaceutical ingredients which are within the scope of sound medical judgment suitable for use in pharmaceutical products.

The term "stable" herein relates to a physical and chemical stability of the pharmaceutical composition of simvastatin or salt thereof.

The term "surfactant" herein related to agent used to disperse the simvastatin or salt thereof and facilitate the dispersion of simvastatin into the water without incorporating air.

The term "low hygroscopic excipient" refers to substance which enables formulation to maintain low water content thus reducing degradation of simvastatin.

The term "Impurity A" refers to (3R,SR}-7-[(1S,2S,6R8S,8aR)-8-[(2,2-dimethylbutanoyl)oxy]-2,6-dimethyl-1,2,6,7,8,8a-hexahydronaphthalen-1-yl]-3,5-dihydroxyheptanoic acid (tenivastatin).

The term "Impurity B" refers to (1S,3R,7S,8S,8aR)-8-[2-[(2R,4R)-4-(acetyloxy)-6-oxotetrahydro-2H-pyran-2-yl]ethyl]-3,7-dimethyl-1,2,3,7,8,8a-hexahydronaphthalen-1-yl 2,2-dimethylbutanoate.

The term "Impurity C" refers to (1S,3R,7S,8S,8aR)-7-dimethyl-8-[2-[(2R)-6-oxo-3,6-dihydro-2Hpyran-2-yl]ethyl].1,2,3,7,8,8a-hexahydronaphthalen-1-yl2,2-dimethylbutanoate.

The term "Impurity D" refers to 2R,4R)-2-[2-[(1S,2S,6R,8S,8aR)-8-[(2,2-dimethylbutanoyl)oxy)-2,6-dimethyl-1,2,6,7,8,8a-hexahydronaphthalen-1-yl)ethyl)-6-oxotetrahydro-2H-pyran-4-yl (3R,5R)-7-[(1S,2S,6R,8S,8aR)-8-[(2,2-dimethylbutanoyl)-oxy]-2,6-dimethyl-1,2,6,7,8,8a-hexahydronaphthalen]-yl]-3,5-dihydroxyheptanoate.

Another aspect of the invention discloses a pharmaceutical composition for oral administration in the form of an aqueous suspension comprising about 0.3% w/v to about 0.9% w/v of simvastatin or salt thereof, and about 0.05% w/v to about 0.2% w/v of surfactant, wherein the composition has a pH in the range of about 3.5 to about 4.0 or in the range of about 3.5 to about 4.5.

The salts of simvastatin may comprise salts with hydrohalic acid such as hydrochloride, hydrobromide and the like; salts with inorganic acid such as sulfate, nitrate, phosphate and the like; salts with organic acid such as acetate, propionate, hydroxyacetate, 2-hydroxypropionate, pyruvate, malonate, succinate, maleate, fumarate, dihydroxyfumarate, oxalate, benzoate, cinnamate, salicylate, methanesulfonate, ethanesulfonate, benzenesulfonate, p-toluenesulfonate, cyclohexylsulfamate, 4-aminosalicylate and the like; salts with alkaline earth metals like sodium, calcium; and the like can be mentioned.

The pH of the composition can be maintained or achieved by pH modifying agents such as sodium hydroxide or hydrochloric acid. The pH may be about 3.5 to about 4, or about 3.7 to about 4, or 3.7 to 3.9, or 3.7, or 3.8 or 3.9.

The surfactant is selected from partial and fatty acid esters and ethers of polyhydric alcohols such as of glycerol, sorbitol and the like; polysorbates such as polysorbate 20, polysorbate 40, polysorbate 60 and polysorbate 80; sodium dodecyl sulfate (sodium lauryl sulfate), lauryl dimethyl amine oxide, cetyltrimethylammonium bromide (CTAB), polyethoxylated alcohols polyoxyethylene sorbitan, octoxynol; N, N - dimethyldodecylamine-N-oxide, hexadecyltrimethylammonium bromide, polyoxyl 10 lauryl ether, polyoxyl castor oil, nonylphenol ethoxylate, lecithin, methylbenzethonium chloride, and mixture thereof. In one embodiment, the surfactant may be polysorbate. In another embodiment, the polysorbate may be polysorbate 80. In another embodiment, the polysorbate 80 is present in about 0.11% w/v of the composition.

The pharmaceutically acceptable excipient is selected from preservative, buffer, suspending agent, antioxidant, sweetener, flavour and mixture thereof.

The preservative is one or more hydroxybenzoates which are selected from methyl hydroxybenzoate, ethyl hydroxybenzoate, propyl hydroxybenzoate, and mixtures thereof. The concentration of methyl hydroxybenzoate ranges from about 0.05% w/v to about 0.20% w/v. The concentration of ethyl hydroxybenzoate ranges from about 0.025% w/v to about 0.035% w/v. The concentration of propyl hydroxybenzoate ranges from about 0.01% w/v to about 0.20% w/v.

The buffer is used to maintain the pH and hence the stability of the compositions over an appropriate period of time. The buffer is selected from citric acid monohydrate, sodium citrate, glycine, tris, bicine, sodium phosphate, sodium dihydrogen phosphate and mixtures thereof. The combination of citric acid monohydrate and sodium citrate (citrate buffer) may be used as a buffer. In one embodiment, the buffer is present in a concentration of about 1% w/v to about 3% w/v of the composition.

The suspending agent is selected from aluminium magnesium silicate, sodium carmellose, methyl cellulose, hydroxypropylmethylcellulose, sodium alginate, xanthan gum, and mixtures thereof. The xanthan gum may be used as suspending agent and present in about 0.05% w/v to about 1% w/v, about 0.05% w/v to about 0.5% w/v, or about 0.08% w/v of the composition. The aluminium magnesium silicate may be used as suspending agent and present in about 0.05% w/v to about 7.5% w/v, or about 0.72% w/v of the composition. The composition may comprise combination of aluminium magnesium silicate and xanthan gum as suspending agent and the weight ratio of aluminium magnesium silicate and xanthan gum ranges from about 1:1 to about 15:1, or about 1:5 to about 12:1, or about 9:1.

The low hygroscopic excipient may be present in high quantity that is about 70% w/v to about 90% w/v, or about 80% w/v to about 90% w/v, or about 85% w/v of the composition. The high concentration of low hygroscopic excipient enables the composition to have low water content and reduces degradation of simvastatin. The low hygroscopic excipient may include excipients like maltitol.

The sweeteners may be sucrose, saccharine, e.g. sodium saccharin, acesulfame K or aspartame. Any suitable flavour may be used in the present invention and suitable flavours are well known in the art. Suitable processing aids which serve to aid dissolution of the one or more preservatives and to aid in dispersing the one or more suspending agents include glycols, such as propylene glycol.

The composition remains physically and chemical stable over appropriate period of time. In one embodiment, the composition remains stable for at least 1 month, or 2 months, or 3 months or 6 months, or 12 months, or 24 months, or 36 months when stored at about 25°C and about 60% (RH) relative humidity.

In another embodiment, the composition remains stable for at least 1 month, or 2 months, or 3 months or 6 months, when stored at about 40°C and about 75% relative humidity.

One embodiment discloses a pharmaceutical composition for oral administration in the form of an aqueous suspension comprising about 0.4% w/v, or about 0.5 % w/v, or about 0.6 % w/v, or about 0.7 % w/v, or about 0.8 % w/v of simvastatin or salt thereof, and about 0.06% w/v, or about 0.07% w/v, or about 0.08% w/v, or about 0.09% w/v, or about 0.10% w/v, or about 0.11% w/v, or about 0.12% w/v, or about 0.14% w/v, or about 0.16% w/v, or about 0.18% w/v of polysorbate 80, wherein the composition has a pH in the range of about 3.5 to about 4.0 or in the range of about 3.5 to about 4.5, and wherein the composition remains stable for at least 1 month, or 2 months, or 3 months or 6 months, or 12 months, or 24 months, or 36 months when stored at about 25°C and about 60% relative humidity.

Another embodiment discloses a pharmaceutical composition for oral administration in the form of an aqueous suspension comprising about 0.4% w/v, or about 0.5% w/v, or about 0.6 % w/v, or about 0.7% w/v, or about 0.8% w/v of simvastatin or salt thereof, and about 0.06% w/v, or about 0.07% w/v, or about 0.08% w/v, or about 0.09% w/v, or about 0.10% w/v, or about 0.11% w/v, or about 0.12% w/v, or about 0.14% w/v, or about 0.16% w/v, or about 0.18% w/v of polysorbate 80, wherein the composition has a pH in the range of 3.7 to 3.9, and wherein the composition remains stable for at least 1 month, or 2 months, or 3 months or 6 months, or 12 months, or 24 months, or 36 months when stored at about 25°C and about 60% relative humidity.

One embodiment discloses a pharmaceutical composition for oral administration in the form of an aqueous suspension comprising: (a) about 0.3% to about 0.9% w/v of simvastatin or salt thereof, (b) about 0.05% w/v to about 0.2 % w/v of surfactant, (c) about 0.05% w/v to about 7.5% w/v of aluminium magnesium silicate, (d) about 0.05% w/v to about 0.5% w/v of xanthan gum, (e) about 1% w/v to about 4% w/v of propylene glycol, (f) about 70% w/v to about 90% w/v of liquid maltitol and (g) a buffer, wherein the composition has a pH in the range of 3.5 to 4.0 or in the range of 3.5 to 4.5, and wherein the aluminium magnesium silicate and xanthan gum is present in a weight ratio of about 1:1 to about 15:1.

Another embodiment discloses a pharmaceutical composition for oral administration in the form of an aqueous suspension comprising: (a) about 0.3% to about 0.9% w/v of simvastatin or salt thereof, (b) about 0.05% w/v to about 0.2 % w/v of polysorbate 80, (c) about 0.05% w/v to about 7.5% w/v of aluminium magnesium silicate, (d) about 0.05% w/v to about 0.5% w/v of xanthan gum, (e) about 1% w/v to about 4% w/v of propylene glycol, (f) about 70% w/v to about 90% w/v of liquid maltitol and (g) a buffer, wherein the composition has a pH in the range of 3.7 to 3.9, and wherein the aluminium magnesium silicate and xanthan gum is present in a weight ratio of about 1:1 to about 15:1.

Another embodiment discloses a pharmaceutical composition for oral administration in the form of an aqueous suspension comprising: (a) about 0.3% to about 0.9% w/v of simvastatin or salt thereof, (b) about 0.05% w/v to about 0.2 % w/v of polysorbate 80, (c) about 0.05% w/v to about 7.5% w/v of aluminium magnesium silicate, (d) about 0.05% w/v to about 0.5% w/v of xanthan gum, (e) about 1% w/v to about 4% w/v of propylene glycol, (f) about 70% w/v to about 90% w/v of liquid maltitol and (g) a buffer, wherein the composition has a pH in the range of 3.7 to 3.9 and the composition contains less than about 1% of Impurity A when stored at about 25°C and about 60% RH for a period of at least 3 months.

Further embodiment discloses a pharmaceutical composition for oral administration in the form of an aqueous suspension comprising: (a) about 0.4% of simvastatin or salt thereof, (b) about 0.11% w/v of polysorbate 80, (c) about 0.72% w/v of aluminium magnesium silicate, (d) about 0.08% w/v of xanthan gum, (e) about 3.50% w/v of propylene glycol, (f) about 70% w/v to about 90% w/v of liquid maltitol and (g) a buffer, wherein the composition has a pH in the range of 3.7 to 3.9 and the composition contains less than about 1% of Impurity A when stored at about 25°C and about 60% RH for a period of at least 3 months.

Yet another embodiment discloses a pharmaceutical composition for oral administration in the form of an aqueous suspension comprising: (a) about 0.8% of simvastatin or salt thereof, (b) about 0.11% w/v of polysorbate 80, (c) about 0.72% w/v of aluminium magnesium silicate, (d) about 0.08% w/v of xanthan gum, and (e) about 3.50% w/v of propylene glycol, wherein the composition has a pH in the range of 3.7 to 3.9.

Yet another embodiment discloses a pharmaceutical composition for oral administration in the form of an aqueous suspension comprising: (a) about 0.4% or about 0.8% w/v of simvastatin or salt thereof, (b) about 0.11% w/v of polysorbate 80, (c) about 0.72% w/v of aluminium magnesium silicate, (d) about 0.08% w/v of xanthan gum, (e) about 3.50% w/v of propylene glycol, (f) about 85% w/v of maltitol and a buffer, wherein the composition has a pH in the range of 3.7 to 3.9.

One embodiment discloses a pharmaceutical composition for oral administration in the form of an aqueous suspension comprising about 0.3% w/v to about 0.9% w/v of simvastatin or salt thereof, and about 0.05% w/v to about 0.2% w/v of surfactant, wherein the composition has a pH in the range of about 3.5 to about 4, for use in the treatment of hypercholesterolemia.

One embodiment discloses a process of preparing a pharmaceutical composition for oral administration in the form of an aqueous suspension comprises steps of:
(i) adding and mixing the pharmaceutically acceptable excipient(s) in water to obtain a mixture,
(ii) separately adding the surfactant into water with stirring to obtain a dispersion,
(iii) adding gradually simvastatin or salt thereof to the dispersion of step (ii) with mixing,
(iv) adding the dispersion of step (iii) into the mixture of step (i) with mixing to obtain dispersion, and
(v) finally adjusting a volume with water and the pH to obtain the pharmaceutical composition for oral administration in the form of an aqueous suspension.

One embodiment discloses a process of preparing a pharmaceutical composition for oral administration in the form of an aqueous suspension comprising about 0.3% w/v to about 0.9% w/v of simvastatin or salt thereof, about 0.05% w/v to about 0.2% w/v of surfactant and a pharmaceutically acceptable excipient(s), wherein the composition has a pH in the range of about 3.5 to about 4.0 or in the range of about 3.5 to about 4.5, wherein the process comprises steps of:
(i) adding and mixing the pharmaceutically acceptable excipient(s) in water to obtain a mixture,
(ii) separately adding the surfactant into water with stirring to obtain a dispersion,
(iii) adding gradually simvastatin or salt thereof to the dispersion of step (ii) with mixing,
(iv) adding the dispersion of step (iii) into the mixture of step (i) with mixing to obtain dispersion, and
(v) finally adjusting a volume with water and the pH to obtain the pharmaceutical composition for oral administration in the form of an aqueous suspension.

Another embodiment discloses a process of preparing a pharmaceutical composition for oral administration in the form of an aqueous suspension comprising about 0.3% w/v to about 0.9% w/v of simvastatin or salt thereof, about 0.05% w/v to about 0.2% w/v of polysorbate 80 and a pharmaceutically acceptable excipient(s), wherein the composition has a pH in the range of about 3.5 to about 4.0 or in the range of about 3.5 to about 4.5, wherein the process comprises steps of:
(i) adding and mixing the pharmaceutically acceptable excipient(s) in water to obtain a mixture,
(ii) separately adding polysorbate 80 into water with stirring to obtain a dispersion,
(iii) adding gradually simvastatin or salt thereof to the dispersion of step (ii) with mixing,
(iv) adding the dispersion of step (iii) into the mixture of step (i) with mixing to obtain dispersion, and
(v) finally adjusting a volume with water and the pH to obtain the pharmaceutical composition for oral administration in the form of an aqueous suspension.

Another embodiment discloses a process of preparing a stable pharmaceutical composition for oral administration in the form of an aqueous suspension comprising about 0.3% w/v to about 0.9% w/v of simvastatin or salt thereof, about 0.05% w/v to about 0.2% w/v of polysorbate 80 and a pharmaceutically acceptable excipient(s), wherein the composition has a pH in the range of about 3.5 to about 4.0 or in the range of about 3.5 to about 4.5, wherein the process comprises steps of:
(i) adding and mixing the pharmaceutically acceptable excipient(s) in water to obtain a mixture,
(ii) separately adding polysorbate 80 into water with stirring to obtain a dispersion,
(iii) adding gradually simvastatin or salt thereof to the dispersion of step (ii) with mixing,
(iv) adding the dispersion of step (iii) into the mixture of step (i) with mixing to obtain dispersion, and
(v) finally adjusting a volume with water and the pH to obtain the pharmaceutical composition for oral administration in the form of an aqueous suspension, wherein the composition remains stable for at least 1 month, or 2 months, or 3 months or 6 months, or 12 months, or 24 months, or 36 months when stored at about 25°C and about 60% relative humidity.

Still another embodiment discloses a process of preparing a stable pharmaceutical composition for oral administration in the form of an aqueous suspension comprising about 0.3% w/v to about 0.9% w/v of simvastatin or salt thereof, about 0.05% w/v to about 0.2% w/v of polysorbate 80 and a pharmaceutically acceptable excipient(s), wherein the composition has a pH in the range of 3.7 to about 3.9, wherein the process comprises steps of:
(i) adding and mixing the pharmaceutically acceptable excipient(s) in water to obtain a mixture,
(ii) separately adding polysorbate 80 into water with stirring to obtain a dispersion,
(iii) adding gradually simvastatin or salt thereof to the dispersion of step (ii) with mixing,
(iv) adding the dispersion of step (iii) into the mixture of step (i) with mixing to obtain dispersion, and
(v) finally adjusting a volume with water and the pH to obtain the pharmaceutical composition for oral administration in the form of an aqueous suspension, wherein the composition remains stable for at least 1 month, or 2 months, or 3 months or 6 months, or 12 months, or 24 months, or 36 months when stored at about 25°C and about 60% relative humidity.

Yet another embodiment discloses a process of preparing a stable pharmaceutical composition for oral administration in the form of an aqueous suspension comprising about 0.4% w/v or about 0.8% w/v of simvastatin or salt thereof, about 0.11% w/v of polysorbate 80 and a pharmaceutically acceptable excipient(s), wherein the composition has a pH in the range of 3.7 to about 3.9, wherein the process comprises steps of:
(i) adding and mixing the pharmaceutically acceptable excipient(s) in water to obtain a mixture,
(ii) separately adding polysorbate 80 into water with stirring to obtain a dispersion,
(iii) adding gradually simvastatin or salt thereof to the dispersion of step (ii) with mixing,
(iv) adding the dispersion of step (iii) into the mixture of step (i) with mixing to obtain dispersion, and
(v) finally adjusting a volume with water and the pH to obtain the pharmaceutical composition for oral administration in the form of an aqueous suspension, wherein the composition remains stable for at least 1 month, or 2 months, or 3 months or 6 months, or 12 months, or 24 months, or 36 months when stored at about 25°C and 60% relative humidity.

While the invention has been described in term of its specific embodiments, certain modification and equivalents will be apparent to those skilled in the art and are intended to be included within the scope of the appended claims.

### EXAMPLES

### EXAMPLE 1: Pharmaceutical aqueous suspensions comprising 20mg/5ml of simvastatin

**Table 1**

| SN | Ingredients | Compositions(20mg/5ml) | | | |
|---|---|---|---|---|---|
| | | A | B | C | D |
| | | %w/v | %w/v | %w/v | %w/v |
| 1 | Simvastatin | 0.40 | 0.40 | 0.40 | 0.40 |
| 2 | Aluminium magnesium silicate | 0.72 | 0.70 | 0.71 | 0.68 |
| 3 | Xanthan gum | 0.08 | 0.1 | 0.5 | 1.0 |
| 4 | Polysorbate 80 | 0.11 | 0.05 | 0.08 | 0.15 |
| 5 | Glycerol | 1.26 | 1.26 | 1.26 | 1.26 |
| 6 | Liquid maltitol | 85.0 | 85.0 | 85.0 | 85.0 |
| 7 | Propylene glycol | 3.50 | 4.00 | 3.00 | 4.50 |
| 8 | Methyl parahydroxybenzoate | 0.14 | 0.12 | 0.10 | 0.13 |
| 9 | Ethyl parahydroxybenzoate | 0.032 | 0.030 | 0.035 | 0.031 |
| 10 | Propyl parahydroxybenzoate | 0.022 | 0.020 | 0.018 | 0.025 |
| 11 | Citric acid monohydrate | 1.60 | 1.60 | 1.60 | 1.60 |
| 12 | Sodium citrate | 1.07 | 1.07 | 1.07 | 1.07 |
| 13 | Orange flavour | 0.025 | 0.025 | 0.025 | 0.025 |
| 14 | Sodium hydroxide | q.s to pH 3.7 -3.8 | | | |
| 15 | Hydrochloric acid | q.s to pH 3.7 -3.8 | | | |
| 16 | Purified water | q.s. to final batch size | | | |

### Manufacturing process:

1. Approximately 85% of the batch volume of purified water was added to the manufacturing vessel.
2. Aluminium magnesium silicate was added and mixed until dispersed.
3. Xanthan gum was dispersed with stirring in the glycerol and added immediately to the manufacturing vessel with mixing until fully hydrated.
4. Liquid maltitol was added with stirring until dispersed.
5. Propylene glycol was heated to approximately 45-55°C and methyl parahydroxybenzoate was added with stirring until dissolved.
6. Ethyl parahydroxybenzoate was added to the solution from Step 5 with stirring until dissolved.
7. Propyl parahydroxybenzoate was added to the solution from Step 6 with stirring until dissolved.
8. The preservative solution from Step 7 was added to the manufacturing vessel with stirring.
9. Citric acid monohydrate was added to the manufacturing vessel with stirring until dissolved.
10. Sodium citrate was added with stirring until dissolved.
11. Approximately 10% of the batch volume of purified water was heated to approximately 30-40°C and added with stirring to the polysorbate 80 until dispersed.
12. The polysorbate 80 solution was cooled to < 25°C.
13. Simvastatin was gradually added with stirring until a smooth dispersion was obtained.
14. Simvastatin dispersion was added to the batch and mixed until a smooth dispersion was obtained.
15. The orange flavour was added to the batch with stirring.
16. Purified water was added to adjust the batch weight to approximately 98% of the final batch weight and the pH was adjusted with pH modifying agents.
17. Purified water was added to adjust the batch weight to a final weight.
18. The suspension was filtered through a nylon 600µm mesh screen.
19. The suspension was filled into 150ml amber glass bottles and sealed with 28mm white-push and turn tamper evident caps.

### EXAMPLE 2: Pharmaceutical aqueous suspensions comprising 40mg/5ml of simvastatin

**Table 2**

| SN | Ingredients | Compositions (40mg/5ml) | | | |
|---|---|---|---|---|---|
| | | A | B | C | D |
| | | %w/v | %w/v | %w/v | %w/v |
| 1 | Simvastatin | 0.80 | 0.80 | 0.80 | 0.80 |
| 2 | Aluminium magnesium silicate | 0.72 | 0.70 | 0.71 | 0.68 |
| 3 | Xanthan gum | 0.08 | 0.1 | 0.5 | 1.0 |
| 4 | Polysorbate 80 | 0.11 | 0.05 | 0.08 | 0.15 |
| 5 | Glycerol | 1.26 | 1.26 | 1.26 | 1.26 |
| 6 | Liquid maltitol | 85.0 | 85.0 | 85.0 | 85.0 |
| 7 | Propylene glycol | 3.50 | 4.00 | 3.00 | 4.50 |
| 8 | Methyl parahydroxybenzoate | 0.14 | 0.12 | 0.10 | 0.13 |
| 9 | Ethyl parahydroxybenzoate | 0.032 | 0.030 | 0.035 | 0.031 |
| 10 | Propyl parahydroxybenzoate | 0.022 | 0.020 | 0.018 | 0.025 |
| 11 | Citric acid monohydrate | 1.60 | 1.60 | 1.60 | 1.60 |
| 12 | Sodium citrate | 1.07 | 1.07 | 1.07 | 1.07 |
| 13 | Orange flavour | 0.025 | 0.025 | 0.025 | 0.025 |
| 14 | Sodium hydroxide | q.s to pH 3.7 -3.8 | | | |
| 15 | Hydrochloric acid | q.s to pH 3.7 -3.8 | | | |
| 16 | Purified water | q.s. to final batch size | | | |

### Manufacturing process:

The Example 2 compositions were prepared as per the process described in Example 1.

### EXAMPLE 3: Stability studies of Example 1 compositions and Comparative example.

The purpose of this stability study is to provide stability data for oral suspension as disclosed in Example 1 (Simvastatin 20mg/5mL) and Example 2 (Simvastatin 40mg/5mL) of WO2007/125339 A1 and Example 1A and Example 2A aqueous suspensions filled into 150mL amber glass bottles.

Eight 150mL bottles of Example 1A and Example 2A oral suspensions were stored at about 25°C and about 60% RH (relative humidity) and about 40°C and about 75% RH for a period of 3 months. Initially and after 3 months storage at each condition samples were tested for appearance, pH, and assay of simvastatin and impurity profile. Two 150mL bottles of Example 1 (Simvastatin 20mg/5mL) and Example 2 (Simvastatin 40mg/5mL) oral suspension of WO2007/125339 A1 were also stored at about 25°C and about 60% RH and about 40°C about 75% RH for a period of 3 months. Initially and after 3 months storage at each condition, samples were tested for appearance, pH, and assay of simvastatin and impurity profile.

**Table 3**

| Product | Example 1A | | Simvastatin oral suspension; 20mg/5mL Example 1 of WO2007/125339 A1(Comparative example A) | |
|---|---|---|---|---|
| | Test Condition: About 25°C and about 60% RH | | | |
| Test | Initial | 3 months | Initial | 3 months |
| Appearance | White to off-white, opaque, homogenous suspension | | | |
| pH | 3.83 | 3.84 | 6.82 | 6.83 |
| Assay of simvastatin (mg/5mL) | 19.46 | 19.53 | 19.04 | 19.45 |
| Related substances (%)- Impurity A | 0.89 | 0.93 | 1.38 | 2.11 |

| Test Condition: About 40°C and about 75% RH | | | | |
|---|---|---|---|---|
| Test | Initial | 3 months | Initial | 3 months |
| Appearance | White to off-white, opaque, homogenous suspension | | | |
| pH | 3.83 | 3.98 | 6.82 | 6.85 |
| Assay of simvastatin (mg/5mL) | 19.46 | 19.13 | 19.04 | 18.49 |
| Related substances (%)- Impurity A | 0.89 | 1.08 | 1.38 | 4.72 |

**Table 4**

| Product | Example 2A | | Simvastatin oral suspension; 40mg/5mL Example 2 of WO2007/125339 A1 (Comparative example B) | |
|---|---|---|---|---|
| | Test Condition: About 25°C and about 60% RH | | | |
| Test | Initial | 3 months | Initial | 3 months |
| Appearance | White to off-white, opaque, homogenous suspension | | | |
| pH | 3.81 | 3.84 | 7.01 | 7.02 |
| Assay of simvastatin (mg/5mL) | 39.36 | 38.54 | 38.75 | 38.73 |
| Related substances (%)-Impurity A | 0.48 | 0.47 | 1.19 | 1.57 |

| Test Condition: About 40°C and about 75% RH | | | | |
|---|---|---|---|---|
| Test | Initial | 3 months | Initial | 3 months |
| Appearance | White to off-white, opaque, homogenous suspension | | | |
| pH | 3.81 | 3.92 | 7.01 | 7.01 |
| Assay of simvastatin (mg/5mL) | 39.36 | 38.16 | 38.75 | 37.55 |
| Related substances (%)-Impurity A | 0.48 | 0.55 | 1.09 | 4.72 |

As evident from Table 3 and 4 that, in Example 1 and Example 2 compositions of WO2007/125339 A1, the assay of simvastatin showed no significant change at about 25°C and about 60% RH. After 3 months at about 40°C and about 75% RH a slight drop in assay by approximately 3% was noted. There was an increase impurity after 3 months at both conditions. This was mainly due to increase in impurity A (Tenivastatin) which is a hydrolysis product. Samples stored at about 25°C and about 60% RH showed increased in impurity A by approximately 0.6%, while samples stored at about 40°C and about 75% RH increased by 3.4%. In invention compositions, the assay of simvastatin showed no significant change at about 25°C and about 60% RH. After 3 months at about 40°C and about 75% RH a slight drop in assay by approximately 3% was noted in the Example 2A product. There was a small but insignificant increase in impurity A of approximately 0.2% in the samples after 3 months at about 40°C and about 75% RH storage.

### EXAMPLE 4: Comparative Example (Composition with and without low hygroscopic agent)

**Table 5**

| SN | Ingredients | Example 4 | Comparative Example C |
|---|---|---|---|
| | | %w/v | %w/v |
| 1 | Simvastatin | 0.80 | 0.80 |
| 2 | Aluminium magnesium silicate | 0.72 | 0.72 |
| 3 | Xanthan gum | 0.08 | 0.08 |
| 4 | Polysorbate 80 | 0.108 | 0.108 |
| 5 | Glycerol | 1.263 | 1.263 |
| 6 | Liquid maltitol | 85.0 | -- |
| 7 | Propylene glycol | 3.50 | 3.50 |
| 8 | Methyl parahydroxybenzoate | 0.15 | 0.15 |
| 9 | Ethyl parahydroxybenzoate | 0.03 | 0.03 |
| 10 | Propyl parahydroxybenzoate | 0.02 | 0.02 |
| 11 | Citric acid monohydrate | 1.60 | 1.60 |
| 12 | Sodium citrate | 1.07 | 1.07 |
| 13 | Orange flavour | 0.03 | 0.03 |
| 14 | Sodium hydroxide | q.s to pH 3.7 -3.8 | |
| 15 | Hydrochloric acid | q.s to pH 3.7 -3.8 | |
| 16 | Purified water | Qs to final volume | |

### Manufacturing process:

The Example 4 compositions were prepared as per the process described in Example 1.

**Table 6**

| Product | Example 4 | | Comparative Example C | |
|---|---|---|---|---|
| | Test Condition: About 25°C and about 60% RH | | | |
| Test | Initial | 3 months | Initial | 3 months |
| Appearance | White to off-white, opaque, homogenous suspension | | | |
| pH | 3.72 | 3.71 | 3.78 | 3.72 |
| Assay of simvastatin (mg/5mL) | 39.87 | 39.42 | 38.72 | 38.10 |
| Related substances (%)-Impurity A | 0.48 | 0.47 | 1.19 | 1.57 |

## Claims

1. A pharmaceutical composition for oral administration in the form of an aqueous suspension comprising: (a) about 0.3 % w/v to about 0.9% w/v of simvastatin or salt thereof; (b) a low hygroscopic excipient; and (c) a buffer, wherein the composition has a pH in the range of about 3.5 to about 4.0, and the composition contains less than about 1% of Impurity A when stored at about 25°C and about 60% RH for a period of at least 3 months.

2. The pharmaceutical composition of claim 1, wherein the simvastatin or salt thereof is present in about 0.4 % w/v of the composition.

3. The pharmaceutical composition of claim 1, wherein the simvastatin or salt thereof is present in about 0.8 % w/v of the composition.

4. The pharmaceutical composition of claim 1, wherein the composition has a pH in the range of 3.6 to 3.8.

5. The pharmaceutical composition of claim 1, wherein the low hygroscopic excipient is present in an amount ranging from about 70% w/v to about 90% w/v of the composition .

6. The pharmaceutical composition of claim 1, wherein the low hygroscopic excipient is maltitol.

7. The pharmaceutical composition of claim 6, wherein the maltitol is present from about 80 to about 90 % by weight of the composition.

8. The pharmaceutical composition of claim 6, wherein the maltitol is present in an amount of about 85% by weight of the composition.

9. The pharmaceutical composition of claim 1, wherein the composition further comprises a pharmaceutically acceptable excipient.

10. The pharmaceutical composition of claim 9, wherein the pharmaceutically acceptable excipient is selected from preservative, surfactant, suspending agent, sweetener, flavour and mixture thereof.

11. The pharmaceutical composition of claim 10, wherein the surfactant comprises polysorbate.

12. The pharmaceutical composition of claim 11, wherein the polysorbate is polysorbate 20 or polysorbate 80.

13. A pharmaceutical composition for oral administration in the form of an aqueous suspension comprising: (a) about 0.4 % w/v of simvastatin or salt thereof, (b) about 0.11% w/v of polysorbate 80, (c) about 0.72% w/v of aluminium magnesium silicate, (d) about 0.08% w/v of xanthan gum, (e) about 3.50% w/v of propylene glycol, (f) about 70% w/v to about 90% w/v of liquid maltitol and (g) a buffer, wherein the composition has a pH in the range of 3.5 to 3.9, and the composition contains less than about 1% of Impurity A when stored at about 25°C and about 60% RH for a period of at least 3 months.

14. A pharmaceutical composition for oral administration in the form of an aqueous suspension comprising: (a) about 0.8% w/v of simvastatin or salt thereof, (b) about 0.11% w/v of polysorbate 80, (c) about 0.72% w/v of aluminium magnesium silicate, (d) about 0.08% w/v of xanthan gum, (e) about 3.50% w/v of propylene glycol, (f) about 70% w/v to about 90% w/v of liquid maltitol and (g) a buffer, wherein the composition has a pH in the range of 3.5 to 3.9, and the composition contains less than about 1% of Impurity A when stored at about 25°C and about 60% RH for a period of at least 3 months.

15. A process of preparing a pharmaceutical composition of claims 1-12, wherein said process comprises steps of:
(i) adding and mixing the pharmaceutically acceptable excipient(s) in water to obtain a mixture,
(ii) separately adding the surfactant into water with stirring to obtain a dispersion,
(iii) adding gradually simvastatin or salt thereof to the dispersion of step (ii) with mixing,
(iv) adding the dispersion of step (iii) into the mixture of step (i) with mixing to obtain dispersion, and
(v) finally adjusting a volume with water and the pH to obtain the pharmaceutical composition in the form of an aqueous suspension.
